Europäisches Patentamt

European Patent Office

Office européen des brevets

(19)

(11) Publication number: **0 436 469 B1**

# EUROPEAN PATENT SPECIFICATION

(12)

(45) Date of publication of patent specification: **08.02.95**

(51) Int. Cl.6: **A61K 38/27**, //(A61K38/27, 31:565,31:66,38:23)

(21) Application number: **90811032.3**

(22) Date of filing: **27.12.90**

(54) **Composition and method for the treatment of osteoporosis in mammals.**

(30) Priority: **03.01.90 US 460416**

(43) Date of publication of application:
**10.07.91 Bulletin 91/28**

(45) Publication of the grant of the patent:
**08.02.95 Bulletin 95/06**

(84) Designated Contracting States:
**AT BE CH DE DK ES FR GB GR IT LI LU NL SE**

(56) References cited:
**EP-A- 0 289 314**
**EP-A- 0 318 184**

**Acta Endocrinologica, vol. 106, p. 167, 1985**

**Calcified Tissue International, vol. 35, p. 578, 1983**

**Rote Liste, Editio Cantor, Aulendorf/Württ., 1988, nos. 65002-65006, 75086**

**Eur. J. Biochem, vol. 190, p. 445, 1990**

**The New England Journal of Medicine, p. 906, 1987**

(73) Proprietor: **CIBA-GEIGY AG**
**Klybeckstrasse 141**
**CH-4002 Basel (CH)**

(72) Inventor: **Bürk, Robert R., Dr.**
**Sichelweg 55**
**CH-4103 Bottmingen (CH)**

Rank Xerox (UK) Business Services
(3.10/3.09/3.3.3)

**Description**

Field of the Invention

The present invention concerns a method for the manufacture of a medicament for the treatment of patients having osteoporosis in which such patients exhibit decreased bone mineral density and patients substantially at risk of developing such decreased bone mineral density through the administration of insulin-like growth factor I (IGF-I) and pharmaceutical compositions therefore.

Background of the Invention

Osteoporosis encompasses a broad range of clinical syndromes having varying etiologies. In postmenopausal women, for example, two distinct types of osteoporosis have been identified. Type I osteoporosis occurs mainly in the early postmenopausal period from about age 50-65. It is characterized by excessive resorption, primarily in trabecular bone. Vertebral fractures are common and if given prior to significant bone loss, treatment which decreases or prevents bone resorption (such as estrogen or calcitonin) is considered effective therapy.

Type II osteoporosis (senile osteoporosis) occurs essentially in all aging women and, to a lesser extent, in men. It is characterized by proportionate loss of cortical and trabecular bone. Here decreased bone formation plays a major role, if not a more important role than increased bone resorption. Fractures of the hip are characteristic of this type.

Currently approved therapeutic agents for osteoporosis are antiresorptives. As such, they are not as effective in patients with established osteoporosis of either type (decreased bone density with fractures of the vertebrae and/or hip), or in patients with Type II osteoporosis. In addition, the most accepted preventive agent for osteoporosis currently in use is estrogen therapy, which is not really an acceptable therapeutic agent for women with a history of breast cancer or endometrial cancer or for men with osteoporosis.

Insulin-like Growth Factor I (IGF-I) is a 70 amino acid peptide belonging to a family of compounds under the class name somatomedins. IGF-I is normally produced in the liver and released for binding to carrier proteins in the plasma (Schwander et al, Endocrin. 113 (1):297-305, 1983), which bound form is inactive.

IGF-I has been demonstrated to specifically bind to receptors on rat osteoblast-like bone cells (Bennett et al, Endocrin. 115 (4): 1577-1583, 1984).

IGF-I infused into rats has been shown to result in markedly greater increases in body weight gain compared to controls, with increases in tibial epiphyseal width and thymidine incorporation into costal cartilage (Nature 107: 16-24, 1984) and directly stimulate osteoblasts to result in a greater number of functional osteoblasts. IGF-I is also mentioned as the vehicle through which growth hormone's effects on bone is mediated in Simpson, Growth Factors Which Affect Bone, Physiol. 235, TIBS, 12/84.

Nevertheless, it is important to note that the foregoing pre-clinical studies were conducted with fetal or newborn rat cells. It is highly likely that such "young" cells are more responsive to IGF-I (as well as other influences) than older cells, especially those in the elderly with established osteoporosis or those with drug or environmentally induced defects leading to reduced bone density.

Surprisingly, IGF-I has now been found to be useful in the manufacture of a medicament for the treatment of osteoporosis in mammals exhibiting decreased bone mineral density and those exposed to drugs or environmental conditions which tend to result in bone density reduction and potentially to an osteoporosis condition.

Accordingly, an object of the present invention is to provide a method for the manufacture of a medicament for the treatment of osteoporosis in mammals exhibiting decreased bone mineral density and preventing osteoporosis due to bone mineral density reduction in patients who are clinically prone to such bone mineral density reductions.

Another object of the invention is to provide pharmaceutical compositions useful in achieving the foregoing object.

Summary of the Invention

The present invention is directed to a method for the manufacture of a medicament and composition both useful in the treatment of osteoporosis in patients demonstrating bone mineral density reductions and preventing such osteoporosis in patients prone thereto by administering to a patient having such osteoporosis or prone thereto an effective amount of IGF-I.

Detailed Description of the Invention

The present invention concerns a method for the manufacture of a medicament for the treatment of osteoporosis in a mammal having reduced bone mineral density or prevention thereof in a mammal prone thereto comprising administering to said mammal in need thereof an effective amount for said treatment or prevention, respectively, of IGF-I or a biologically active fragment thereof, especially the treatment or prevention of such osteoporosis in humans.

IGF-I is a naturally occurring protein and can be obtained from a number of sources. Preferably, IGF-I from the same species, (or its synthetic twin) as is being treated therewith is employed but IGF-I from one species may be used to treat another species if the immune response elicited is slight or nonexistent. In addition, fragments of IGF-I having IGF-I activity, particularly IGF-I antiosteoporosis activity, are also suitable for employment and unless the context of the disclosure clearly indicates otherwise, IGF-I as used herein is intended to include such active fragments. Where weights of IGF-I are presented, that weight of IGF-I and an approximately equipotent amount of active fragments is intended unless the text explicitly states otherwise. Where no type of IGF-I is indicated, reference is to human IGF-I (meaning the structure, not the species source), unless the reasonable reading of the text indicates otherwise.

IGF-I can be synthetically produced, chemically or by recombinant techniques, although recombinant preparation is preferred. One such recombinant technique is disclosed in EP 123,228.

An effective amount of IGF-I is an amount sufficient to slow, stop, or reverse the bone mineral density reduction rate in a patient exhibiting bone mineral density reduction. In the normal healthy 20-25 year old population bone mineral density in the spine (using dual photon densitometry) typically is in the range of 0.85 to 1.9 g/cm$^3$, usually 0.9 to 1.85 and most often 1.0 to. 1.8; and in the mid radius and distal radius it is typically 0.7-1.4, usually 0.75-1.3, and most often 0.8-1.2 g/cm$^3$. Exemplary non-limiting normal ranges are given e.g. in *Mayo Clin. Proc.,* Dec. 1985, Vol. 60, p. 827-835 and in *Orthopedic Clinics of North America,* Vol. 16, No. 3, July 1985, p. 557-568. Norms using other techniques will also be apparent from the literature and will grow as general experience with the experience in such techniques. Of course, it is to be remembered that different sub-populations have different norms in bone mineral density. For example caucasian women typically differ in this parameter from caucasian men as well as from black women, oriental women and women of other racial types. It is also important to remember that the current invention is directed to treating those with bone mineral density which is (a) totally below either the normal bone mineral density range for the population generally or for the patient sub-population or (b) below 1.0 g/cm$^3$ or (c) below the fracture threshold (approximately 2 standard deviations below the mean bone mass for the population at age 35). The fracture threshold for the spine for example is defined as the bone mineral value below which 90% of all patients with one or more compression fractures of the spine are found (See Mayo Clin. Proc., Dec. 1985, Vol 60, p. 829-830). In addition, anyone who demonstrated a statistically significant reduction in bone density over a previous measurement, regardless of where that patient is in the typical ranges above, is a patient to whom the present invention treatment is directed. Statistical significance in this context will vary with the technique employed to measure bone mineral density, as well as with the sensitivity of the instruments used. However, with instrumentation and techniques generally available in 1988, a 1 or 2% change in bone mineral density from the earliest measurement to the most recent is not considered statistically significant. Still as techniques and equipment improve, persons of ordinary skill in the field of bone density measurement will revise downward the maximum percent change which is not considered statistically significant.

Current bone mineral density measurement techniques include dual energy radiography, quantitative computerized tomography, single photon densitometry, and dual photon densitometry. These techniques will be well known to those of ordinary skill in the art; however, descriptions thereof can be found in: *Mayo Clin. Proc.,* Dec. 1985, Vol. 60, p. 827-835; *Orthopedic Clinics of North America,* Vol. 16, No. 3, July 1985, p. 557-568; *Hologic QDR™ − 1,000 Product Literature; Annals of Internal Medicine,* 1984, 100: p. 908-911; and *Clinical Physiol* 4:343, 1984.

The reference *Mayo Clin. Proc.,* Dec. 1985, Vol. 60, p. 827-835, for example, gives following data which themselves are based on data from Riggs BL, Wahner HW, Dunn WL, Mazess RB, Offord KP, Melton LJ III, "Differential changes in bone mineral density of the appendicular and axial skeleton with aging: relationship to spinal osteoporosis."J. Clin. Invest. 67:328-335, 1981:

- Bone mineral density (BMD) in spine (measured with use of dual-photon absorptiometry), midradius, and distal radius (measured with use of single-photon absorptiometry) in 76 women with osteoporosis in comparison with age- and sex-adjusted normal range (105 women). Spinal measurements results in the best distinction of patients with osteoporosis from normal subjects because this disease primarily affects trabecular bone of the spine.

- Fracture threshold for spinal bone mineral (horizontal line) superimposed on normal range and values for 76 patients with osteoporosis. With progressing age, values of increasing numbers of normal subjects are below the fracture threshold. Fracture threshold is approximately two standard deviations below mean bone mass at age 35 years.

The reference *Orthopedic Clinics of North America,* Vol. 16, No. 3, July 1985, p. 557-568 shows for example following:

- Normal male values for vertebral cancellous mineral content by QCT, using a cubic regression with 95 percent confidence intervals.
- Normal female values for vertebral cancellous mineral content by QCT, using a cubic regression with 95 per cent confidence intervals. An accelerated loss is observed after menopause.
- The accuracy of single-energy QCT shown for vertebral specimens (preserved in sodium azide) from 11 patients (10 men and 1 women), ages 40 to 90 years.
- Values for men with idiopathic osteoporosis and spinal fractures compared with the normal male curve. A fracture threshold at approximately 110 mg/cm$^3$ is observed.
- Idiopathic osteoporotic male values showing larger decrement from normal for vertebral mineral QCT than for mean peripheral cortical mineral by radiogrammetry and photon absorptiometry.

The invention concerns the treatment of osteoporosis wherein the reduced bone mineral density is characterized as being at or below the lower 10th percentile of the general population, preferentially at or below the lower 10th percentile of the racial, sexual, or both racial and sexual subpopulations, of mammals of the same species between the ages of 1 1/2 times and 2 times reproductive maturity for said species. Said mammal preferentially is a human being and said 1 1/2 times to 2 times reproductive maturity is the age bracket of 20-25.

Notwithstanding, the lack of statistical significance in a particular result, any bone mineral density reduction should be followed for further reductions, which cumulatively may be significant.

The invention in particular concerns the treatment of osteoporosis with a reduction in bone mineral density in excess of 2%, more preferably 4%, most preferably 6% of any prior bone mineral density measurement in said mammal.

Usually, an effective amount of IGF-I, when given parenterally (intravenously, subcutaneously, intramuscularly, etc.), is between 2.5 $\mu$g/Kg/day up to 500 $\mu$g/Kg/day, preferably 5 $\mu$g/Kg/day up to 400 $\mu$g/Kg/day, more preferably 10 $\mu$g/Kg/day up to 340 $\mu$g/Kg/day, even more preferably 40 $\mu$g/Kg/day up to 250 $\mu$g/Kg/day, still more preferably 60 $\mu$g/Kg/day up to 180 $\mu$g/Kg/day. When given continuously, such effective amount may be given in two or three doses spread over time such as by i.v. drip or subcutaneous injection(s) with the total daily dose being spread across the portion or the entire administration period. Typical continuous dosing is in the range of 0.1 $\mu$g/Kg/hour up to 25 $\mu$g/Kg/hour, preferably 1 $\mu$g/Kg/hour up to 14 $\mu$g/Kg/hour, more preferably 2.5 $\mu$g/Kg/hour up to 7.5 $\mu$g/Kg/hour, although wider ranges of "continuous" administration amounts will be apparent to those of ordinary skill. When given by subcutaneous injection, it is most preferably administered from 3 times/week up to 3 times a day, preferably twice a week up to once or twice daily.

The specific dosage for a particular patient, of course, has to be adjusted to the degree of response, the route of administration, the individual weight and general condition of the patient to be treated, and is finally dependent upon the judgement of the treating physician.

The invention also concerns pharmaceutical preparations for use in the present invention comprising an effective amount of IGF-I or an active fragment thereof together with a pharmaceutically and parenterally acceptable carrier or adjuvant. Compositions having an approximately 6 day supply typically contain from 0.1 mg to 15 mg, preferably 1 mg to 13 mg, more preferably 3 mg to 10 mg, most preferably 5 mg-10 mg of IGF-I. The liquid carriers are typically sterile water, approximate physiologic saline optionally together with 0.01 M acetic acid, 5% aqueous dextrose, etc.; preferably sterile water, physiologic saline, or 5% aqueous dextrose.

The carriers and adjuvants may be solid or liquid and may be organic or inorganic. The active compound and the compositions of the invention are preferably used in the form of preparations or infusions for parenteral (subcutaneous, intramuscular, or intravenous) administration. Such solutions are preferably isotonic aqueous solutions or suspensions which can be prepared before use, for example reconstituted from a lyophilised preparation. The pharmaceutical preparations may be sterilized and/or contain adjuvants, for example preservatives, stabilizers, wetting agents, emulsifiers, solubilizers, tonicity regulating salts, and/or buffers. Other adjuvants will of course be apparent to the ordinarily skilled formulation chemist.

The present pharmaceutical preparations may contain further pharmacologically active or otherwise pharmaceutically valuable substances. Accordingly, the invention concerns also a pharmaceutical composition for the treatment of osteoporosis in mammals exhibiting bone mineral density reduction or prevention thereof in mammals prone thereto comprising a combination of an effective amount of IGF-I or a biologically active fragment thereof with a bone antiresorptive effective amount of a bone antiresorptive active compound or a pharmaceutically acceptable salt thereof, and optionally a pharmaceutically acceptable carrier. Said bone antiresorptives are for example estrogen, preferentially conjugated estrogen or estradiol, further bisphosphonates, particularly 3-aminopropy-1-hydroxy-1,1-bisphosphonate and also in particular calcitonin, such as salmon calcitonin, chicken calcitonin, eel calcitonin, rat calcitonin, porcine calcitonin, ovine calcitonin, bovine calcitonin an most preferentially human calcitonin.

The pharmaceutical compositions of the invention are prepared from their constituent parts by techniques known in the art, for example lyophilization, dissolution, reconstitution, and suspension techniques, among others known to those of ordinary skill. They typically contain from 0.1% to 100% of active ingredient, especially in the case of solutions - 0.2% to 20% active ingredient and especially in the case of a lyophilizate - up to 100% of active ingredient.

The invention concerns for example a pharmaceutically acceptable formulation of a parenteral combination of therapeutic bone antiresorptive doses of IGF-I as defined hereinbefore, e.g. in a concentration of 0.2 up to 20 mg/ml, preferably 10 mg/ml, and of a calcitonin as defined hereinbefore, e.g. in a concentration of 0.02 up to 2 mg/ml, preferably 0.5 mg/ml. Said formulation constitutes for example a freeze-dried preparation in vials or ampoules incorporating a suitable bulking agent (carrier). Such agents are mannitol, e.g. in a concentration of 20 mg/ml, lactose, trehalose or another pharmaceutically acceptable carrier. The lyophilized mixture can be reconstituted by dissolving in a parenteral aqueous solvent, such as sterile water for injection, 5% dextrose solution, or physiological saline optionally together with O.1 M acetic acid, or the like. Preferred are sterile water, 5% dextrose solution, or physiological saline.

The invention also concerns the use of IGF-I for the production of a pharmaceutical preparation for the treatment of osteoporosis in mammals exhibiting bone mineral density reduction or prevention thereof in mammals prone thereto. Said pharmaceutical preparation may also comprise a combination of an effective amount of IGF-I or a biologically active fragment thereof with a bone antiresorptive effective amount of a bone antiresorptive active compound, and optionally a pharmaceutically acceptable carrier.

The invention further concerns the pharmaceutical compositions and methods as herein described, particularly with reference to the accompanying examples.

Having fully described the present invention, the following non-limiting Examples are presented to more clearly set forth the invention without limiting it.

Examples 1-3: Dry ampoules of IGF-I

Sterile, filtered 1% (w/v) aqueous solution of IGF-I is added, in the amount indicated to the respective dry ampoules the solution is then lyophilized to result in the dry ampoules to be reconstituted shortly before use with the indicated amount of sterile water, physiologic saline, 0.1 M acetic acid, or 5% aqueous dextrose. Each vial is sufficient for a 6 day course of treatment for the intended patient.

|                        | Ex 1 | Ex 2 | Ex 3  |
|------------------------|------|------|-------|
| ampoule size           | 5 ml | 8 ml | 50 ml |
| IGF-I fill volume      | 1 ml | 5 ml | 30 ml |
| Reconstitution Volume  | 1 ml | 5 ml | 30 ml |

Example 4: Stimulation of bone formation in rats with ovariectomy-induced osteoporosis

Two groups of 8 female rats of 130 - 150 g body weight are ovariectomized by the dorsal route. Six weeks after the operation, treatment is started. Group 1 forms the control group receiving the vehicle, group 2 receives 2.5 mg/kg/day IGF-I, which is dissolved in 0.01 M acetic acid plus 0.9% (w/v) sodium chloride. The IGF-I and the vehicle are administered subcutaneously by osmotic mini-pump. The animals are treated during 3 weeks. At autopsy, both femurs are prepared free of connective tissue and their length is determined using a capiller. The amount of trabecular and cortical bone is determined according to Gunnes-Hey, M. and Hock, J.M. (1984), Metab. Bone Dis. and Rel. Res. 5:177-181. Briefly, the femurs are cut in half at the middiaphysis using a dental saw. The proximal halves are discarded. With a scalpel the epiphysis of the distal half is cut off, the bone is split with a scalpel into saggital halves. The marrow is flushed out with water. With a dental curette the trabecular bone is scratched out of both halves, combined and put into 5% TCA. The 2 pieces of the remaining cortical bone are also combined and put into a separate tube with 5% TCA. After standing 16 h at room temperature, the TCA extract is removed and used for the determination of calcium. The remaining demineralized matrix is washed successively with ethanol and methylenechloride and dried in the vacuum. After determination of the dry weight, the matrix is hydrolyzed with 6M HCl at 120 °C for 5 h. In the hydrolysate hydroxyproline is determined by a standard colorimetric assay according to Jamall, I.S. et al. (1981) Analyt. Biochem. 112:70 - 75. The results are shown in the following table:

| Parameter | Group 1 | Group 2 | $\delta$ | p |
|---|---|---|---|---|
| Length of femur (mm) | 28.31±0.17 | 28.03±0.17 | -1% | NS |
| Trabecular bone: | | | | |
| calcium (mg) | 1.6±0.2 | 2.3±0.2 | +39% | <0.05 |
| dry weight (mg) | 1.8±0.3 | 2.4±0.2 | +38% | <0.05 |
| hydroxyproline (µg) | 160±21 | 231±22 | +44% | <0.05 |
| Cortical bone: | | | | |
| calcium (mg) | 22.6±0.5 | 22.9±0.7 | +1% | NS |
| dry weight (mg) | 67.4±2.3 | 73.6±3.8 | +9% | NS |
| hydroxyproline (µg) | 3594±55 | 3727±97 | +1% | NS |
| $\delta$ = Difference between IGF-I and control group  NS = not significant  p = significance in the Student's T-test | | | | |

The data in the table show that IGF-I increases the amount of trabecular bone in osteoporotic rats as measured by the content of calcium, the dry weight and the content of hydroxyproline. The increase is approximately 40% and statistically significant (p<0.05). This increase is not due to an enhanced longitudinal growth, as the length of the femurs in the control group and in the IGF-I group is the same. The amount of cortical bone is not affected by IGF-I.

In conclusion, these data show that IGF-I is able to increase the trabecular bone mass and thus to stimulate bone formation in ovariectomy-induced osteoporosis in rats.

Example 5: Production of a combination preparation comprising IGF-I and Calcitonin

1 ml of aqueous solution of IGF-I (1% w/v), calcitonin (0.05% w/v) and as bulking agent pyrogen-free mannitol (2% w/v) is sterile filtered through a 0.2 µm filter and filled into sterile vials. The solution is then lyophilized to result in dry ampoules to be reconstituted shortly before use with 1 ml amount of sterile water, physiologic saline, 0.1 M acetic acid, or 5% aqueous dextrose. In analog processes, 20 mg of lactose or trehalose is used as bulking agent.

**Claims**
**Claims for the following Contracting States : AT, BE, CH, DE, DK, FR, GB, IT, LI, LU, NL, SE**

1. A pharmaceutical composition for the treatment of osteoporosis in mammals exhibiting bone mineral density reduction or prevention thereof in mammals prone thereto comprising a combination of an effective amount of IGF-I or a biologically active fragment thereof with a bone antiresorptive effective amount of a bone antiresorptive active compound, and optionally a pharmaceutically acceptable carrier.

2. The composition of claim 1 wherein said composition is a parenteral composition.

3. The composition of claim 1 wherein said mammal is a human being.

4. The composition of claim 1 wherein said IGF-I and said IGF-I active fragment of IGF-I are, respectively, human IGF-I and an IGF-I active fragment of human IGF-I.

5. The composition of claim 1 wherein said IGF-I is of natural origin.

6. The composition of claim 1 wherein said IGF-I or said IGF-I active fragment of IGF-I is of synthetic origin.

7. The composition of claim 1 wherein said IGF-I or said IGF-I active fragment of IGF-I is manufactured via recombinant technology.

8. The composition of claim 1 wherein said bone antiresorptive active compound is selected from an estrogen, a calcitonin, and a hydroxy-alkyl bisphosphonate.

9. The composition of claim 8 wherein said estrogen is a conjugated estrogen or estradiol, said calcitonin is a human calcitonin, and said hydroxyalkyl-bisphosphonate is 3-amino-propyl-1-hydroxy-1,1-bisphosphonate or a pharmaceutically acceptable salt thereof.

10. The use of IGF-I or a biologically active fragment thereof for the production of a pharmaceutical composition for the treatment of osteoporosis in mammals exhibiting bone mineral density reduction or prevention thereof in mammals prone thereto.

11. The use of claim 10 wherein said mammal is a human being.

12. The use of claim 10 wherein said IGF-I is chemically the same as natural IGF-I from the same species as is said mammal receiving said treatment or preventive administration therewith and said IGF-I active fragment of IGF-I is a fragment which is found in natural IGF-I from the same species as is said mammal receiving said treatment or preventive administration therewith.

13. The use of claim 10 wherein said IGF-I is naturally occurring IGF-I or IGF-I active fragment of IGF-I.

14. The use of claim 10 wherein said IGF-I is synthetically produced IGF-I or an IGF-I active fragment of IGF-I.

15. The use of claim 10 wherein said IGF-I or IGF-I active fragment of IGF-I is produced by recombinant techniques.

16. The use of claim 10 wherein said pharmaceutical preperation is for parenteral administration

17. The use of claim 10 wherein said reduction in bone mineral density in said mammal is in excess of 2% of any prior bone mineral density measurement in said mammal.

18. The use of claim 17 wherein said reduction in bone density in said mammal is in excess of 4% of any previous bone density measurement in said mammal.

19. The use of claim 18 wherein said reduction in bone mineral density in said mammal is in excess of 6% of any prior bone mineral density measurement in said mammal.

20. The use of claim 10 wherein said reduced bone mineral density is characterized as being at or below the lower 10th percentile of the general population of mammals of the same species between the ages of 1 1/2 times and 2 times reproductive maturity for said species.

21. The use of claim 20 wherein said reduced bone mineral density is characterized as being at or below the lower 10th percentile of the racial, sexual, or both racial and sexual subpopulations of mammals of the same species between the ages of 1 1/2 times and 2 times reproductive maturity for said species.

22. The use of claim 20 wherein said mammal is a human being and said 1 1/2 times to 2 times reproductive maturity is the age bracket of 20-25.

23. The use of claim 10 for the production of a pharmaceutical composition for the treatment of osteoporosis with IGF-I, wherein said pharmaceutical composition is administered in conjunction with a bone anti-resorptive amount of a bone anti-resorptive active compound.

7

24. The use of claim 23 for the production of a pharmaceutical composition for the treatment of osteoporosis with IGF-I, wherein said pharmaceutical composition is administered substantially simultaneously with said bone anti-resorptive compound.

25. The use of claim 23 for the production of a pharmaceutical composition for the treatment of osteoporosis with IGF-I, wherein said pharmaceutical composition and said bone anti-resorptive compound are administered by different routes.

26. The use of claim 10 wherein said pharmaceutical composition is a combination of IGF-I or a biologically active fragment thereof with a bone antiresorptive effective amount of a bone antiresorptive active compound, and optionally a pharmaceutically acceptable carrier.

**Claims for the following Contracting States : ES, GR**

1. The use of IGF-I for the production of a pharmaceutical composition for for the treatment of osteoporosis in mammals exhibiting bone mineral density reduction or prevention thereof in mammals prone thereto.

2. The use of claim 1 wherein said composition is a parenteral composition.

3. The use of claim 1 wherein said mammal is a human being.

4. The use of claim 1 wherein said IGF-I is chemically the same as natural IGF-I from the same species as is said mammal and said IGF-I active fragment of IGF-I is a fragment which is found in natural IGF-I from the same species as is said mammal.

5. The use of claim 1 wherein said IGF-I and said IGF-I active fragment of IGF-I are, respectively, human IGF-I and an IGF-I active fragment of human IGF-I.

6. The use of claim 1 wherein said IGF-I is of natural origin.

7. The use of claim 1 wherein said IGF-I or said IGF-I active fragment of IGF-I is of synthetic origin.

8. The use of claim 1 wherein said IGF-I or said IGF-I active fragment of IGF-I is manufactured via recombinant technology.

9. The use of claim 1 wherein said pharmaceutical composition is a combination of IGF-I or a biologically active fragment thereof with a bone antiresorptive effective amount of a bone antiresorptive active compound, and optionally a pharmaceutically acceptable carrier.

10. The use of claim 9 wherein said bone antiresorptive active compound is selected from an estrogen, a calcitonin, and a hydroxy-alkyl bisphosphonate.

11. The use of claim 9 wherein said estrogen is a conjugated estrogen or estradiol, said calcitonin is a human calcitonin, and said hydroxy-alkyl-bisphosphonate is 3-amino-propyl-1-hydroxy-1,1-bisphosphonate or a pharmaceutically acceptable salt thereof.

12. The use of claim 1 wherein said reduction in bone mineral density in said mammal is in excess of 2% of any prior bone mineral density measurement in said mammal.

13. The use of claim 12 wherein said reduction in bone density in said mammal is in excess of 4% of any previous bone density measurement in said mammal.

14. The use of claim 12 wherein said reduction in bone mineral density in said mammal is in excess of 6% of any prior bone mineral density measurement in said mammal.

15. The use of claim 1 wherein said reduced bone mineral density is characterized as being at or below the lower 10th percentile of the general population of mammals of the same species between the ages of 1 1/2 times and 2 times reproductive maturity for said species.

**16.** The use of claim 1 wherein said reduced bone mineral density is characterized as being at or below the lower 10th percentile of the racial, sexual, or both racial and sexual subpopulations of mammals of the same species between the ages of 1 1/2 times and 2 times reproductive maturity for said species.

**17.** The use of claim 1 wherein said mammal is a human being and said 1 1/2 times to 2 times reproductive maturity is the age bracket of 20-25.

**18.** The use of claim 1 for the production of a pharmaceutical composition for the treatment of osteoporosis with IGF-I, wherein said pharmaceutical composition is administered in conjunction with a bone anti-resorptive amount of a bone anti-resorptive active compound.

**19.** The use of claim 18 for the production of a pharmaceutical composition for the treatment of osteoporosis with IGF-I, wherein said pharmaceutical composition is administered substantially simultaneously with said bone anti-resorptive compound.

**20.** The use of claim 18 for the production of a pharmaceutical composition for the treatment of osteoporosis with IGF-I, wherein said pharmaceutical composition and said bone anti-resorptive compound are administered by different routes.

**Patentansprüche**
**Patentansprüche für folgende Vertragsstaaten : AT, BE, CH, DE, DK, FR, GB, IT, LI, LU, NL, SE**

**1.** Pharmazeutische Zusammensetzung zur Behandlung von Osteoporose bei Säugern, die eine Verringerung der Knochenmineraldichte zeigen, oder zur Verhütung davon bei Säugern, die dazu neigen, umfassend eine Kombination aus einer wirksamen Menge an IGF-I oder eines biologisch aktiven Fragments davon mit einer gegen die Knochenresorption wirksamen Menge einer gegen die Knochenresorption aktiven Verbindung und gegebenenfalls einen pharmazeutisch verträglichen Träger.

**2.** Zusammensetzung nach Anspruch 1, wobei die Zusammenserzung eine parenterale Zusammensetzung ist.

**3.** Zusammensetzung nach Anspruch 1, wobei der Säuger ein Mensch ist.

**4.** Zusammensetzung nach Anspruch 1, wobei der IGF-I und das IGF-I-aktive Fragment von IGF-I jeweils menschlicher IGF-I bzw. ein IGF-I-aktives Fragment von menschlichem IGF-I sind.

**5.** Zusammensetzung nach Anspruch 1, wobei der IGF-I natürlichen Ursprungs ist.

**6.** Zusammensetzung nach Anspruch 1, wobei der IGF-I oder das IGF-I-aktive Fragment von IGF-I synthetischen Ursprungs sind.

**7.** Zusammensetzung nach Anspruch 1, wobei der IGF-I oder das IGF-I-aktive Fragment von IGF-I durch die rekombinante Technik hergestellt sind.

**8.** Zusammensetzung nach Anspruch 1, wobei die gegen die Knochenresorption wirksame Verbindung aus Östrogen, Calcitonin und Hydroxyalkylbisphosponat ausgewählt ist.

**9.** Zusammensetzung nach Anspruch 8, wobei das Östrogen ein konjugiertes Östrogen oder Östradiol ist, das Calcitonin menschliches Calcitonin ist, und das Hydroxyalkylbisphonat 3-Aminopropyl-1-hydroxy-1,1-bisphosphonat oder ein pharmazeutisch verträgliches Salz davon ist.

**10.** Verwendung von IGF-I oder eines biologisch aktiven Fragments davon zur Herstellung einer pharmazeutischen Zusammensetzung zur Behandlung von Osteoporose bei Säugern, die eine Verringerung der Knochenmineraldichte zeigen, oder zur Verhütung davon, bei Säugern, die dazu neigen.

**11.** Verwendung nach Anspruch 10, wobei der Säuger ein Mensch ist.

**12.** Verwendung nach Anspruch 10, wobei der IGF-I chemisch gleich dem natürlichen IGF-I aus der gleichen Art wie der Säuger, der die Behandlung oder die vorbeugende Verabreichung damit erhält, ist, und wobei das IGF-I-aktive Fragment von IGF-I ein Fragment ist, das in natürlichem IGF-I der gleichen Art wie der Säuger, der die Behandlung oder vorbeugende Verabreichung damit erhält, gefunden wird.

**13.** Verwendung nach Anspruch 10, wobei der IGF-I ein natürlich vorkommender IGF-I oder ein natürlich vorkommendes IGF-I-aktives Fragment von IGF-I ist.

**14.** Verwendung nach Anspruch 10, wobei der IGF-I synthetisch erzeugter IGF-I oder ein synthetisch erzeugtes IGF-I-aktives Fragment von IGF-I ist.

**15.** Verwendung nach Anspruch 10, wobei der IGF-I oder das IGF-I-aktive Fragment durch rekombinante Techniken produzierter IGF-I ist.

**16.** Verwendung nach Anspruch 10, wobei das pharmazeutische Zusammensetzung der parenteralen Verabreichung dient.

**17.** Verwendung nach Anspruch 10, wobei die Verringerung der Knochenmineraldichte in dem Säuger mehr als 2 % jeder früheren Knochenmineraldichtemessung bei dem Säuger beträgt.

**18.** Verwendung nach Anspruch 17, wobei die Verringerung der Knochenmineraldichte in dem Säuger mehr als 4 % jeder früheren Knochenmineraldichtemessung bei dem Säuger beträgt.

**19.** Verwendung nach Anspruch 18, wobei die Verringerung der Knochenmineraldichte in dem Säuger mehr als 6 % jeder früheren Knochenmineraldichtemessung bei dem Säuger beträgt.

**20.** Verwendung nach Anspruch 10, wobei die verringerte Knochenmineraldichte dadurch gekennzeichnet ist, daß sie bei oder unter dem untersten 10-Percentilwert der allgemeinen Population der Säuger der gleichen Art zwischen dem Alter des 1,5fachen und 2fachen der Reproduktionsreife für die Art liegt.

**21.** Verwendung nach Anspruch 20, wobei die verringerte Knochenmineraldichte dadurch gekennzeichnet ist, daß sie bei oder unter dem untersten 10-Percentilwert der rassenbezogenen, geschlechtsbezogenen oder sowohl rassenbezogenen als auch geschlechtsbezogenen Subpopulationen der Säuger der gleichen Art zwischen dem Alter des 1,5fachen und 2fachen Reproduktionsreife für diese Art liegt.

**22.** Verwendung nach Anspruch 20, wobei der Säuger ein Mensch ist, und das 1,5fache bis 2fache der Reproduktionsreife die Altersspanne von 20 bis 25 ist.

**23.** Verwendung nach Anspruch 10 zur Herstellung einer pharmazeutischen Zusammensetzung zur Behandlung von Osteoporose mit IGF-I, wobei die pharmazeutische Zusammensetzung zusammen mit einer gegen die Knochenresorption wirkenden Menge einer Verbindung, die gegen die Knochenresorption wirkt, verabreicht wird.

**24.** Verwendung nach Anspruch 23 zur Herstellung einer pharmazeutischen Zusammensetzung zur Behandlung von Osteoporose mit IGF-I, wobei die pharmazeutische Zusammensetzung im wesentlichen gleichzeitig mit der Verbindung gegen die Knochenresorption verabreicht wird.

**25.** Verwendung nach Anspruch 23 zur Herstellung einer pharmazeutischen Zusammensetzung zur Behandlung von Osteoporose mit IGF-I, wobei die pharmazeutische Zusammensetzung und die Verbindung gegen die Knochenresorption auf verschiedenen Wegen verabreicht werden.

**26.** Verwendung nach Anspruch 10, wobei die pharmazeutische Zusammensetzung eine Kombination aus IGF-I oder einem biologisch aktiven Fragment davon mit einer gegen die Knochenresorption wirkenden, effektiven Menge einer Verbindung gegen die Knochenresorption und gegebenenfalls einem pharmazeutisch verträglichen Träger ist.

**Patentansprüche für folgende Vertragsstaaten : ES, GR**

1. Verwendung von IGF-I zur Herstellung einer pharmazeutischen Zusammensetzung zur Behandlung von Osteoporose bei Säugern, die eine Verringerung der Knochenmineraldichte zeigen, oder zur Verhütung davon bei Säugern, die dazu neigen.

2. Verwendung nach Anspruch 1, wobei die Zusammensetzung eine parenterale Zusammensetzung ist.

3. Verwendung nach Anspruch 1, wobei der Säuger ein Mensch ist.

4. Verwendung nach Anspruch 1, wobei der IGF-I chemisch gleich dem natürlichen IGF-I aus der gleichen Art wie der Säuger ist, und das IGF-I-aktive Fragment von IGF-I ein Fragment ist, das in natürlichem IGF-I aus der gleichen Art wie der Säuger gefunden wird.

5. Verwendung nach Anspruch 1, wobei der IGF-I und das IGF-I-aktive Fragment von IGF-I jeweils menschlicher IGF-I bzw. ein IGF-I-aktives Fragment von menschlichem IGF-I sind.

6. Verwendung nach Anspruch 1, wobei der IGF-I natürlichen Ursprungs ist.

7. Verwendung nach Anspruch 1, wobei der IGF-I oder das IGF-I-aktive Fragment von IGF-I synthetischen Ursprungs ist.

8. Verwendung nach Anspruch 1, wobei der IGF-I oder das IGF-I-aktive Fragment von IGF-I durch die rekombinante Technik hergestellt ist.

9. Verwendung nach Anspruch 1, wobei die pharmazeutische Zusammensetzung eine Kombination aus IGF-I oder einem biologisch aktiven Fragment davon mit einer gegen die Knochenresorption wirkenden effektiven Menge einer gegen die Knochenresorption aktiven Verbindung und ggf. einem pharmazeutisch verträglichen Träger ist.

10. Verwendung nach Anspruch 9, wobei die gegen die Knochenresorption aktive Verbindung aus Östrogen, Calcitonin und Hydroxyalkylbisphosphonat ausgewählt ist.

11. Verwendung nach Anspruch 9, wobei das Östrogen ein konjugiertes Östrogen oder Östradiol ist, das Calcitonin ein menschliches Calcitonin ist, und das Hydroxyalkylbisphosphonat 3-Aminopropyl-1-hydroxy-1,1-bis-phosphonat oder ein pharmazeutisch verträgliches Salz davon ist.

12. Verwendung nach Anspruch 1, wobei die Verringerung der Knochenmineraldichte bei dem Säuger mehr als 2 % jeder früheren Knochenmineraldichtemessung bei dem Säuger beträgt.

13. Verwendung nach Anspruch 12, wobei die Verringerung der Knochenmineraldichte in dem Säuger mehr als 4 % jeder früheren Knochenmineraldichtemessung bei dem Säuger beträgt.

14. Verwendung nach Anspruch 12, wobei die Verringerung der Knochenmineraldichte in dem Säuger mehr als 6 % jeder früheren Knochenmineraldichtemessung bei dem Säuger beträgt.

15. Verwendung nach Anspruch 1, wobei die verringerte Knochenmineraldichte dadurch gekennzeichnet ist, daß sie bei oder unter dem untersten 10-Percentilwert der allgemeinen Population der Säuger der gleichen Art zwischen dem Alter des 1,5fachen und 2fachen der Reproduktionsreife für die Art liegt.

16. Verwendung nach Anspruch 1, wobei die verringerte Knochenmineraldichte dadurch gekennzeichnet ist, daß sie bei oder unter dem untersten 10-Percentilwert der rassenbezogenen, geschlechtsbezogenen oder sowohl rassenbezogenen als auch geschlechtsbezogenen Subpopulationen der Säuger der gleichen Art zwischen dem Alter des 1,5fachen und 2fachen Reproduktionsreife für den Säuger liegt.

17. Verwendung nach Anspruch 1, wobei der Säuger ein Mensch ist, und das 1,5fache bis 2fache der Reproduktionsreife die Altersspanne von 20 bis 25 ist.

EP 0 436 469 B1

**18.** Verwendung nach Anspruch 1 zur Herstellung einer pharmazeutischen Zusammensetzung zur Behandlung von Osteoporose mit IGF-I, wobei die pharmazeutische Zusammensetzung zusammen mit einer gegen die Knochenresorption wirkenden Menge einer Verbindung, die gegen die Knochenresorption wirkt, verabreicht wird.

**19.** Verwendung nach Anspruch 18 zur Herstellung einer pharmazeutischen Zusammensetzung zur Behandlung von Osteoporose mit IGF-I, wobei die pharmazeutische Zusammensetzung im wesentlichen gleichzeitig mit der Verbindung gegen die Knochenresorption verabreicht wird.

**20.** Verwendung nach Anspruch 18 zur Herstellung einer pharmazeutischen Zusammensetzung zur Behandlung von Osteoporose mit IGF-I, wobei die pharmazeutische Zusammensetzung und die Verbindung gegen die Knochenresorption auf verschiedenen Wegen verabreicht werden.

**Revendications**

**Revendications pour les Etats contractants suivants : AT, BE, CH, DE, DK, FR, GB, IT, LI, LU, NL, SE**

**1.** Composition pharmaceutique pour le traitement de l'ostéoporose chez des mammifères présentant une diminution de densité de la minéralisation osseuse, ou pour la prévention de l'ostéoporose chez des mammifères sujets à celle-ci, comprenant une association d'une quantité efficace de facteur de croissance de type I proche de l'insuline (IGF-I) ou d'un fragment biologiquement actif de celui-ci, et d'une quantité efficace anti-résorption osseuse d'un composé à activité anti-résorption osseuse, et éventuellement d'un véhicule pharmaceutiquement acceptable.

**2.** Composition selon la revendication 1, caractérisée en ce que ladite composition est une composition parentérale.

**3.** Composition selon la revendication 1, caractérisée en ce que ledit mammifère est un être humain.

**4.** Composition selon la revendication 1, dans laquelle ledit IGF-I et ledit fragment d'IGF-I à activité d'IGF-I sont, respectivement, l'IGF-I humain et un fragment d'IGF-I humain à activité d'IGF-I.

**5.** Composition selon la revendication 1, dans laquelle ledit IGF-I est d'origine naturelle.

**6.** Composition selon la revendication 1, dans laquelle ledit IGF-I ou ledit fragment d'IGF-I à activité d'IGF-I est d'origine synthétique.

**7.** Composition selon la revendication 1, dans laquelle ledit IGF-I ou ledit fragment d'IGF-I à activité d'IGF-I est produit par une technique de recombinaison.

**8.** Composition selon la revendication 1, dans laquelle ledit composé à activité anti-résorption osseuse est choisi parmi un oestrogène, une calcitonine et un hydroxyalkylbiphosphonate.

**9.** Composition selon la revendication 8, dans laquelle ledit oestrogène est un oestrogène conjugué ou l'oestradiol, ladite calcitonine est une calcitonine humaine, et ledit hydroxyalkylbiphosphonate est le 3-amino-propyl-1-hydroxy-1,1-biphosphonate ou un sel pharmaceutiquement acceptable de celui-ci.

**10.** Utilisation de l'IGF-I ou d'un fragment biologiquement actif de celui-ci, pour la préparation d'une composition pharmaceutique pour le traitement de l'ostéoporose chez des mammifères présentant une diminution de la densité de la minéralisation osseuse, ou pour la prévention de l'ostéoporose chez des mammifères sujets à celle-ci.

**11.** Utilisation selon la revendication 10, dans laquelle ledit mammifère est un être humain.

**12.** Utilisation selon la revendication 10, dans laquelle ledit IGF-I est chimiquement identique à l'IGF-I naturel provenant de la même espèce que le mammifère recevant ledit traitement par ou l'administration préventive de celui-ci, et ledit fragment d'IGF-I à activité d'IGF-I est un fragment qui est rencontré dans l'IGF-I naturel provenant de la même espèce que le mammifère recevant ledit traitement par ou l'administration préventive de celui-ci.

12

**13.** Utilisation selon la revendication 10, dans laquelle ledit IGF-I est de l'IGF-I ou un fragment d'IGF-I à activité d'IGF-I existant dans la nature.

**14.** Utilisation selon la revendication 10, dans laquelle ledit IGF-I est de l'IGF-I ou un fragment d'IGF-I à activité d'IGF-I produit synthétiquement.

**15.** Utilisation selon la revendication 10, dans laquelle ledit IGF-I ou fragment d'IGF-I à activité d'IGF-I est produit par des techniques de recombinaison.

**16.** Utilisation selon la revendication 10, dans laquelle ladite composition pharmaceutique est destinée à l'administration parentérale.

**17.** Utilisation selon la revendication 10, dans laquelle ladite diminution de densité de la minéralisation osseuse chez ledit mammifère excède 2 % de toute mesure antérieure de la densité de minéralisation osseuse chez ledit mammifère.

**18.** Utilisation selon la revendication 17, dans laquelle ladite diminution de densité osseuse chez ledit mammifère excède 4 % de toute mesure précédente de la densité osseuse chez ledit mammifère.

**19.** Utilisation selon la revendication 18, dans laquelle ladite diminution de densité de la minéralisation osseuse chez ledit mammifère excède 6 % de toute mesure antérieure de la densité de minéralisation osseuse chez ledit mammifère.

**20.** Utilisation selon la revendication 10, dans laquelle ladite densité réduite de la minéralisation osseuse est caractérisée comme rentrant dans les 10 % inférieurs ou moins de la population générale de mammifères de la même espèce entre les âges représentant $1\frac{1}{2}$ à 2 fois la maturité reproductrice pour ladite espèce.

**21.** Utilisation selon la revendication 20, dans laquelle ladite densité réduite de la minéralisation osseuse est caractérisée comme rentrant dans les 10 % inférieurs ou moins des sous-populations raciales, sexuelles ou à la fois raciales et sexuelles de mammifères de la même espèce entre les âges représentant $1\frac{1}{2}$ et 2 fois la maturité reproductrice pour ladite espèce.

**22.** Utilisation selon la revendication 20, dans laquelle ledit mammifère est un être humain et ladite tranche d'âge représentant $1\frac{1}{2}$ à 2 fois la maturité reproductrice est la tranche d'âge de 20-25 ans.

**23.** Utilisation selon la revendication 10, pour la préparation d'une composition pharmaceutique destinée au traitement de l'ostéoporose par l'IGF-I, dans laquelle ladite composition pharmaceutique est administrée conjointement avec une quantité anti-résorption osseuse d'un composé à activité anti-résorption osseuse.

**24.** Utilisation selon la revendication 23, pour la préparation d'une composition pharmaceutique destinée au traitement de l'ostéoporose par l'IGF-I, dans laquelle ladite composition pharmaceutique est administrée pratiquement en même temps que ledit composé anti-résorption osseuse.

**25.** Utilisation selon la revendication 23, pour la préparation d'une composition pharmaceutique destinée au traitement de l'ostéoporose par l'IGF-I, dans laquelle ladite composition pharmaceutique et ledit composé antirésorption osseuse sont administrés par des voies différentes.

**26.** Utilisation selon la revendication 10, dans laquelle ladite composition pharmaceutique est une association d'IGF-I ou d'un fragment biologiquement actif de celui-ci et d'une quantité efficace anti-résorption osseuse d'un composé à activité anti-résorption osseuse, et éventuellement d'un véhicule pharmaceutiquement acceptable.

**Revendications pour les Etats contractants suivants : ES, GR**

**1.** Utilisation du facteur de croissante de type I proche de l'insuline (IGF-I) pour la préparation d'une composition pharmaceutique destinée au traitement de l'ostéoporose chez des mammifères présentant une diminution de densité de la minéralisation osseuse, ou à la prévention de l'ostéoporose chez des mammifères sujets à celle-ci.

2. Utilisation selon la revendication 1, caractérisée en ce que ladite composition est une composition parentérale.

3. Utilisation selon la revendication 1, caractérisée en ce que ledit mammifère est un être humain.

4. Utilisation selon la revendication 1, dans laquelle ledit IGF-I est chimiquement identique à l'IGF-I naturel provenant de la même espèce que ledit mammifère, et ledit fragment d'IGF-I à activité d'IGF-I est un fragment qui est rencontré dans l'IGF-I naturel provenant de la même espèce que ledit mammifère.

5. Utilisation selon la revendication 1, dans laquelle ledit IGF-I et ledit fragment d'IGF-I à activité d'IGF-I sont, respectivement, l'IGF-I humain et un fragment d'IGF-I humain à activité d'IGF-I.

6. Utilisation selon la revendication 1, dans laquelle ledit IGF-I est d'origine naturelle.

7. Utilisation selon la revendication 1, dans laquelle ledit IGF-I ou ledit fragment d'IGF-I à activité d'IGF-I est d'origine synthétique.

8. Utilisation selon la revendication 1, dans laquelle ledit IGF-I ou ledit fragment d'IGF-I à activité d'IGF-I est produit par une technique de recombinaison.

9. Composition selon la revendication 1, dans laquelle ladite composition pharmaceutique est une association d'IGF-I, ou d'un fragment biologiquement actif de celui-ci, et d'une quantité efficace anti-résorption osseuse d'un composé à activité anti-résorption osseuse, et éventuellement d'un véhicule pharmaceutiquement acceptable.

10. Utilisation selon la revendication 9, dans laquelle ledit composé à activité anti-résorption osseuse est choisi parmi un oestrogène, une calcitonine et un hydroxyalkylbiphosphonate.

11. Utilisation selon la revendication 9, dans laquelle ledit oestrogène est un oestrogène conjugué ou l'oestradiol, ladite calcitonine est une calcitonine humaine, et ledit hydroxyalkylbiphosphonate est le 3-amino-propyl-1-hydroxy-1,1-biphosphonate ou un sel pharmaceutiquement acceptable de celui-ci.

12. Utilisation selon la revendication 1, dans laquelle ladite diminution de densité de la minéralisation osseuse chez ledit mammifère excède 2 % de toute mesure antérieure de densité de la minéralisation osseuse chez ledit mammifère.

13. Utilisation selon la revendication 12, dans laquelle ladite diminution de densité osseuse chez ledit mammifère excède 4 % de toute mesure précédente de la densité osseuse chez ledit mammifère.

14. Utilisation selon la revendication 12, dans laquelle ladite diminution de densité de la minéralisation osseuse chez ledit mammifère excède 6 % de toute mesure antérieure de la densité de minéralisation osseuse chez ledit mammifère.

15. Utilisation selon la revendication 1, dans laquelle ladite densité réduite de la minéralisation osseuse est caractérisée comme rentrant dans les 10 % inférieurs ou moins de la population générale de mammifères de la même espèce entre les âges représentant 1 fois $\frac{1}{2}$ à 2 fois la maturité reproductrice pour ladite espèce.

16. Utilisation selon la revendication 1, dans laquelle ladite densité réduite de la minéralisation osseuse est caractérisée comme rentrant dans les 10 % inférieurs ou moins des sous-populations raciales, sexuelles ou à la fois raciales et sexuelles de mammifères de la même espèce entre les âges représentant 1 $\frac{1}{2}$ et 2 fois la maturité reproductrice pour ladite espèce.

17. Utilisation selon la revendication 1, dans laquelle ledit mammifère est un être humain et ladite tranche d'âge représentant 1 $\frac{1}{2}$ à 2 fois la maturité reproductrice est la tranche d'âge de 20-25 ans.

**18.** Utilisation selon la revendication 1, pour la préparation d'une composition pharmaceutique destinée au traitement de l'ostéoporose par l'IGF-I, dans laquelle ladite composition pharmaceutique est administrée conjointement avec une quantité anti-résorption osseuse d'un composé à activité anti-résorption osseuse.

**19.** Utilisation selon la revendication 18, pour la préparation d'une composition pharmaceutique destinée au traitement de l'ostéoporose par l'IGF-I, dans laquelle ladite composition pharmaceutique est administrée pratiquement en même temps que ledit composé anti-résorption osseuse.

**20.** Utilisation selon la revendication 18, pour la préparation d'une composition pharmaceutique destinée au traitement de l'ostéoporose par l'IGF-I, dans laquelle ladite composition pharmaceutique et ledit composé antirésorption osseuse sont administrés par des voies différentes.